# EUROPEAN PATENT SPECIFICATION

(11) **EP 4 034 540 B1**
(45) Date of publication and mention of the grant of the patent: **08.11.2023**
(21) Application number: 20775623.0
(22) Date of filing: 21.09.2020
(51) Int. Cl.: C07D 493/04, C08L 69/00

(54) **METHOD OF MAKING AND/OR ISOLATING ISOIDIDE, COMPRISING SELECTIVE ESTERIFICATION OF A MIXTURE OF DIANHYDROHEXITOL ISOMERS**
VERFAHREN ZUR HERSTELLUNG UND/ODER ISOLIERUNG VON ISOIDID, UMFASSEND SELEKTIVE VERESTERUNG EINER MISCHUNG VON DIANHYDROHEXITOLISOMEREN
PROCÉDÉ DE FABRICATION ET/OU D'ISOLATION D'ISOIDIDE, COMPRENANT L'ESTÉRIFICATION SÉLECTIVE D'UN MÉLANGE D'ISOMÈRES DE DIANHYDROHEXITOL

(30) Priority: 23.09.2019 EP 19198984
(43) Date of publication of application: 03.08.2022
(73) Proprietor: BASF SE, 67056 Ludwigshafen am Rhein (DE); THE REGENTS OF THE UNIVERSITY OF CALIFORNIA, Oakland, CA 94607-5200 (US)
(72) Inventor: MASCAL, Mark, Oakland, California 94607-5200 (US); SASKA, Jan, Oakland, California 94607-5200 (US); KINDLER, Alois, 67056 Ludwigshafen (DE); ZUEND, Stephan, Fremont, California 94538 (US); DUTTA, Saikat, Oakland, California 94607-5200 (US)
(74) Representative: Eisenführ Speiser
(86) International application number: PCT/EP2020/076254
(87) International publication number: WO 2021/058425

(56) References cited:
- WO-A1-2013/125950
- US-A- 3 023 223
- US-A1- 2012 116 101

## Description

The present invention relates to a method of making and/or isolating isoidide, to a composition comprising a mixture comprising non-esterified isoidide and one or both compounds selected from the group consisting of esterified isosorbide and esterified isomannide and to the use of such composition in a method for making and/or isolating isoidide. The present invention further pertains to the use of a method comprising a step of selective esterification of a mixture of dianhydrohexitol isomers comprising isoidide, for separating isoidide from said mixture of dianhydrohexitol isomers. Moreover, the present invention pertains to a method of making a polymer comprising isoidide monomers or modified isoidide monomers.

Dianhydrohexitols, also sometimes referred to as "isohexides", are bicyclic, oxygen-containing heterocycles formed from two anellated tetrahydrofuran rings. The dianhydrohexitols comprise the compounds isosorbide, isomannide and isoidide. Said dianhydrohexitols can e.g be obtained from twofold dehydration of the sugar alcohols sorbitol, mannitol or iditol, respectively, by processes known in the art.

Isoidide is a compound which has received attention because of its usefulness in industrial synthesis, e.g. as building block for polymers which are derived from renewable resources and/or which are bio-degradable.

Because isosorbide can be made from D-glucose via D-sorbitol as intermediate and isomannide can be made from D-fructose via D-mannitol as intermediate, both, isosorbide and isomannide are usually available in industrial quantities without particular difficulties. However, iditol as starting material for the production of isoidide is not available in technical quantities. Efforts have therefore been made in the past for preparing isoidide in larger amounts, including epimerization methods using isosorbide or isomannide as starting materials.

The following literature deals with certain aspects of modifying dianhydrohexitols:
Document US 4,417,065 describes a process for the preparation of isosorbide 2-nitrate.
Document EP 3 056 496 A1 pertains to a method of making isoidide.
Document US 2012/116101 A1 discloses a method for preparing dianhydrohexitol diester compositions.
Document US 3,023,223 deals with a process for producing isoidide.
Document WO 2013/125950 A1 discloses a method of making isoidide.
P. Stoss et al. in Synthesis 1987, 174-176, report of regioselective acylation of 1,4:3,6-dianhydro-D-glucitol.

In the light of the existing prior art, there is still a need for a more convenient method of making and/or isolating isoidide, more in particular for a method of making and/or isolating isoidide with increased efficiency, including increased yield from a step of isolating isoidide.

Correspondingly, it was a primary object of the present invention to provide a convenient method of making and/or isolating isoidide and a composition for use in such method.

It was another object of the present invention to provide a method of separating isoidide from a mixture of dianhydrohexitol isomers comprising isoidide, by applying a method of selective derivatization of dianhydrohexitol isomers.

In addition, it was a more specific object of the present invention to provide a method of making a polymer comprising isoidide monomers or modified isoidide monomers which are obtained by a more convenient method of making and/or isolating isoidide.

It has now been found that the primary object and other objects of the present invention can be accomplished by a method of making and/or isolating isoidide of formula I 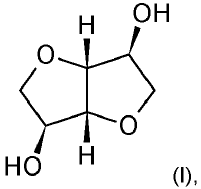 comprising the following steps:
M3) providing or preparing a mixture of compounds of formula II 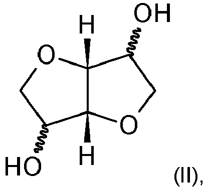 comprising isoidide and one or both compounds selected from the group consisting of isosorbide and isomannide
   and
M4) subjecting the mixture of compounds of formula II from step M3) to conditions of a selective esterification, so that a mixture comprising non-esterified isoidide and one or both compounds selected from the group consisting of esterified isosorbide and esterified isomannide are (or respectively, is,) received,
wherein the selective esterification is performed in the presence of a metal catalyst and comprises reacting the mixture of compounds of formula II with a reagent selected from the group consisting of carboxylic acid anhydrides wherein the carboxylic acid or carboxylic acids forming the carboxylic acid anhydride in each case are selected from the group consisting of carboxylic acids comprising a total number of carbon atoms in the range of from 2 to 8, and
M5) separating non-esterified isoidide from the mixture comprising non-esterified isoidide and one or both compounds selected from the group consisting of esterified isosorbide and esterified isomannide received in step M4),
to obtain and/or isolate non-esterified isoidide.

The invention as well as preferred variants and preferred combinations of parameters, properties and elements thereof are defined in the appended claims. Embodiments not encompassed by the appended claims are provided for reference purposes.

Preferred aspects
details, modifications and advantages of the present invention are also defined and explained in the following description and in the examples shown below.

It has now been found that the method of making and/or isolating isoidide according to the present invention is highly efficient (e.g. in terms of energy consumption and/or cost) and allows a very convenient isolation of isoidide under mild conditions and in high yields from a mixture comprising non-esterified isoidide and esterified dianhydrohexitol isomers.

In particular, it has been found in own experiments that isoidide (under the conditions of the method of making and/or isolating isoidide according to the present invention) is esterified, in particular is acylated, more in particular is acetylated, much slower than isosorbide or isomannide.

In the context of the present invention, the compound isoidide, in accordance with the usual meaning in the technical field, means the compound which is also known under the chemical name "1 ,4:3,6-dianhydro-L-iditol" (CAS RN: 24332-71-6). In the context of the present invention the compound isosorbide, in accordance with the usual meaning in the technical field, means the compound which is also known under the name "D-isosorbide" or under the chemical names "1,4:3,6-dianhydro-D-sorbitol" or "1,4:3,6-dianhydro-D-glucitol" (CAS RN: 652-67-5). In the context of the present invention the compound isomannide, in accordance with the usual meaning in the technical field, means the compound which is also known under the chemical name "1,4:3,6-dianhydro-D-mannitol" (CAS RN: 641-74-7). In the context of the present invention, the terms "isoidide" and "non-esterified isoidide" are used synonymously.

In the context of the present invention, the term "selective esterification" or "stereoselective esterification", in accordance with the usual meaning in the field, means the property of a reactant mixture (here: a mixture of dianhydrohexitol isomers) where a non-stereospecific mechanism (here: non-stereospecific esterification) allows for the formation of multiple products (here: monoester of a dianhydrohexitol, diester of a dianhydrohexitol or non-esterified dianhydrohexitol), but where only one (or only a subset) of the products is favored by factors, such as steric access, that are independent of the reaction mechanism.

In the context of the present invention, the terms "esterified isoidide", "esterified isosorbide", "esterified isomannide" and "esterified dianhydrohexitol" in each case comprise the mono-esterified (specifically: the mono-acylated or mono-acetylated) respective compounds, respectively their monoesters, and the bis-esterified (specifically: the bis-acylated or bis-acetylated) respective compounds, respectively their diesters, as well as mixtures of the respective mono-esterified and bis-esterified compounds. Depending on the particular dianhydrohexitol observed and on the reaction conditions applied for its esterification, the monoester, the diester or a mixture of monoester and diester may result, while either the monoester or the diester may prevail in such mixture ("selective esterification", see above). Where the present text refers to "esterified isosorbide", usually the monoester of isosorbide (specifically: the mono-acylated or mono-acetylated isosorbide) prevails under the conditions of the method of making and/or isolating isoidide according to the present invention. Where the present text refers to "esterified isomannide", usually the diester of isomannide (specifically: the bis-acylated or bis-acetylated isomannide) prevails under the conditions of the method of making and/or isolating isoidide according to the present invention. In certain cases, in particular when the reaction time is short and/or when only relatively small amounts of acylating agent are available, the monoester of isomannide may predominate over other forms of isomannide.

In step M4) as defined above, the mixture comprising non-esterified isoidide and one or both compounds selected from the group consisting of esterified isosorbide and esterified isomannide may also comprise esterified isoidide to a certain extent, as explained and specified in more detail below.

Under a particular aspect, the present invention also pertains to a method of making and/or isolating 1 ,4:3,6-dianhydro-L-iditol, comprising the following steps:
M3a) providing or preparing a mixture of compounds of formula II 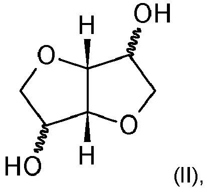 comprising 1,4:3,6-dianhydro-L-iditol and one or both compounds selected from the group consisting of 1,4:3,6-dianhydro-D-sorbitol and 1,4:3,6-dianhydro-D-mannitol
   and
M4a) subjecting the mixture of compounds of formula II from step M3a) to conditions of a selective esterification, so that a mixture comprising non-esterified 1,4:3,6-dianhydro-L-iditol and one or both compounds selected from the group consisting of esterified 1,4:3,6-dianhydro-D-sorbitol and esterified 1,4:3,6-dianhydro-D-mannitol are received.

All aspects of the present invention discussed herein in the context of the steps M3) and M4) of the method of making and/or isolating isoidide according to the present invention as defined herein apply *mutatis mutandis* to the steps M3a) and M4a) of the method of making and/or isolating isoidide according to the present invention, as defined here above.

In the method of making and/or isolating isoidide according to the present invention as defined herein (or a method of making and/or isolating isoidide according to the present invention as described above or below as being preferred) the selective esterification in step M4) (or step M4a)
- is performed in a way to selectively esterify isosorbide and/or isomannide in the presence of isoidide;
   and
- is performed in the presence of a metal catalyst, preferably a metal salt catalyst,
   wherein preferably the metal is selected from the group consisting of calcium, strontium, barium, zinc, cadmium, mercury, indium, thallium, the lanthanides, tin, lead, antimony, bismuth, iron, cobalt and nickel,
   wherein more preferably the metal is selected from the group consisting of barium, mercury, lead and bismuth,
   wherein even more preferably the metal catalyst is or comprises a lead catalyst, preferably a lead salt catalyst, more preferably at least one member of the group consisting of a lead (II) carboxylate and lead (II) oxide, and even more preferably the metal catalyst is or comprises lead (II) acetate;
      and
- comprises reacting the mixture of compounds of formula II with a reagent selected from the group consisting of:
   - carboxylic acid anhydrides wherein the carboxylic acid or carboxylic acids forming the carboxylic acid anhydride in each case (i.e. the carboxylic acid moieties of the carboxylic acid anhydride, of the general formula R^{c}-C(O)- or R^{d}-C(O)-, respectively, where R^{c} and R^{d} stand for the carboxylic acid moieties of the carboxylic acid anhydrides comprising the carbon atoms) are selected from the group consisting of carboxylic acids comprising a total number of carbon atoms in the range of from 2 to 8, preferably of from 2 to 6, wherein preferably the carbon atoms present form (where structurally possible) a branched carbon chain in each case;
      wherein more preferably the carboxylic acids forming the carboxylic acid anhydride in each case are selected from the group consisting of aliphatic carboxylic acids comprising a total number of carbon atoms in the range of from 2 to 8, preferably of from 2 to 6, wherein preferably the carbon atoms present form (where structurally possible) a branched carbon chain in each case; aliphatic carboxylic acids comprising a total number of carbon atoms in the range of from 2 to 8, preferably of from 2 to 6, which are substituted one to three times by fluorine, chlorine, bromine and/or iodine; cycloaliphatic carboxylic acids comprising a total number of carbon atoms in the range of from 4 to 8; cycloaliphatic carboxylic acids comprising a total number of carbon atoms in the range of from 4 to 8 which are substituted one to three times by fluorine, chlorine, bromine and/or iodine; benzoic acid; benzoic acid which is substituted once by fluorine, chlorine, bromine or iodine and nicotinic acid; nicotinic acid which is substituted once by fluorine, chlorine, bromine or iodine;
      wherein even more preferably the carboxylic acid anhydrides are selected from the group consisting of acetic (acid) anhydride, propionic (acid) anhydride, butyric (acid) anhydride, isobutyric (acid) anhydride (i.e. 2-methylpropanoic acid anhydride), pivalic (acid) anhydride (i.e. 2,2-dimethylpropanoic acid anhydride), valeric (acid) anhydride (i.e. pentanoic acid anhydride) and mixtures thereof,
      wherein yet even more preferably the carboxylic acid anhydrides are selected from the group consisting of acetic anhydride, propionic anhydride, butyric anhydride, isobutyric anhydride (i.e. 2-methylpropanoic acid anhydride), pivalic anhydride (i.e. 2,2-dimethylpropanoic acid anhydride) and mixtures thereof;
      wherein preferably the reagent is selected from the group consisting of
   - carboxylic acid anhydrides wherein the carboxylic acid or carboxylic acids forming the carboxylic acid anhydride in each case (i.e. the carboxylic acid moieties of the carboxylic acid anhydride, of the general formula R^{c}-C(O)- or R^{d}-C(O)-, respectively, where R^{c} and R^{d} have the meanings as defined above) are selected from the group consisting of carboxylic acids comprising a total number of carbon atoms in the range of from 2 to 8, preferably of from 2 to 6, wherein preferably the carbon atoms present form (where structurally possible) a branched carbon chain in each case;
      wherein more preferably the carboxylic acids forming the carboxylic acid anhydride in each case are selected from the group consisting of aliphatic carboxylic acids comprising a total number of carbon atoms in the range of from 2 to 8, preferably of from 2 to 6, wherein preferably the carbon atoms present form (where structurally possible) a branched carbon chain in each case; aliphatic carboxylic acids comprising a total number of carbon atoms in the range of from 2 to 8, preferably of from 2 to 6, which are substituted one to three times by fluorine, chlorine, bromine and/or iodine; cycloaliphatic carboxylic acids comprising a total number of carbon atoms in the range of from 4 to 8; cycloaliphatic carboxylic acids comprising a total number of carbon atoms in the range of from 4 to 8 which are substituted one to three times by fluorine, chlorine, bromine and/or iodine; benzoic acid; benzoic acid which is substituted once by fluorine, chlorine, bromine or iodine; nicotinic acid and nicotinic acid which is substituted once by fluorine, chlorine, bromine or iodine;
      wherein even more preferably the carboxylic acid anhydrides are selected from the group consisting of acetic anhydride, propionic anhydride, butyric anhydride, isobutyric anhydride, pivalic anhydride, valeric anhydride and mixtures thereof,
      wherein yet even more preferably the carboxylic acid anhydrides are selected from the group consisting of acetic anhydride, propionic anhydride, butyric anhydride, isobutyric anhydride, pivalic anhydride and mixtures thereof;
      wherein even more preferably the reagent is or comprises acetic anhydride; and/or
   - is performed at a temperature in the range of from -20 °C to 50 °C, preferably of from 10 °C to 50 °C, more preferably of from 15 °C to 45 °C, even more preferably of from 15 °C to 35 °C;
      and/or
   - is performed for a reaction time in the range of from 15 min to 24 hrs, preferably of from 30 min to 10 hrs, more preferably of from 1 h to 5 hrs, yet more preferably of from 1 h to 3 hrs.

In the method of making and/or isolating isoidide according to the present invention as defined here above the selective esterification in step M4) is performed in the presence of a metal catalyst, the metal catalyst is present in homogenous phase or in heterogeneous phase, preferably in homogenous phase. Preferably, the total amount of metal catalyst present in step M4) is in the range of from 0.01 to 10.0 mole-%, more preferably in the range of from 0.05 to 5.0 mole-% and yet more preferably in the range of from 0.1 to 3.0 mole-%, relative to the molar amount of the mixture of compounds of formula II present in step M4).

In the method of making and/or isolating isoidide according to the present invention as defined here above, the reagent for reacting with the mixture of compounds of formula II is or comprises a carboxylic acid anhydride, the anhydride can in each case be symmetrical (i.e. be composed of two acid moieties from the same acid) or unsymmetrical (i.e. be composed of two acid moieties from different acids). For the purposes of the present invention, symmetrical carboxylic acid anhydrides are preferred as reagents for reacting with the mixture of compounds of formula II.

In the method of making and/or isolating isoidide according to the present invention as defined here above the reagent for reacting with the mixture of compounds of formula II is or comprises a carboxylic acid anhydride, the carboxylic acids representing said carboxylic acid anhydride are preferably selected from the group consisting of acetic acid; acetic acid which is substituted one to three times by fluorine, chlorine, bromine and/or iodine; propionic acid; propionic acid which is substituted one to three times by fluorine, chlorine, bromine and/or iodine; butyric acid; butyric acid which is substituted one to three times by fluorine, chlorine, bromine and/or iodine; pivalic acid; pivalic acid which is substituted one to three times by fluorine, chlorine, bromine and/or iodine; valeric acid; valeric acid which is substituted one to three times by fluorine, chlorine, bromine and/or iodine; caproic acid; caproic acid which is substituted one to three times by fluorine, chlorine, bromine and/or iodine; benzoic acid, benzoic acid which is substituted once by fluorine, chlorine, bromine or iodine; nicotinic acid and nicotinic acid which is substituted once by fluorine, chlorine, bromine or iodine.

In the method of making and/or isolating isoidide according to the present invention as defined here above, the total molar amount of the reagent for reacting with the mixture of compounds of formula II present in step M4) is preferably in the range of from 0.5 to 2 moles, more preferably in the range of 0.5 to 1.5 moles, of reagent, relative to the molar amount of the mixture of compounds of formula II present in step M4).

Generally, the mixture of compounds of formula II can be reacted with a reagent as defined above (or a reagent defined above as being preferred) in step M4) (or M4a) of the method of making and/or isolating isoidide according to the present invention according to methods known in the art for esterification of the particular reagent chosen with compounds of formula II (or their mixtures, respectively). As explained above, the stereoselective course of the esterification reaction is in principle independent of the reaction mechanism but will predominantly be controlled by the configuration of the (different) compounds of formula II and can to some extent also be influenced by the nature of the reagent chosen and/or by the nature of the catalyst chosen. Nonetheless, adjusting certain parameters of the reaction in step M4) (or M4a)) can beneficially influence the result of the selective esterification, e.g. in terms of the amount (or ratio) of the desired reaction product received.

The temperature ranges and the reaction time ranges as defined for the method of making and/or isolating isoidide according to the present invention here above (for step M4) or M4a)) characterize favorable parameters which result in a mixture comprising non-esterified isoidide and one or both compounds selected from the group consisting of esterified isosorbide and esterified isomannide (and usually also esterified isoidide) which comprises a high content of non-esterified isoidide and a high content of esterified other dianhydrohexitol isomers.

Said temperature ranges and said reaction time ranges as defined here above (for step M4) or M4a)) are particularly suited for the method of making and/or isolating isoidide according to the present invention wherein the reagent for reacting the mixture of compounds of formula II is or comprises a carboxylic acid anhydride.

Similarly, said temperature ranges and said reaction time ranges as defined here above (for step M4) or M4a)) are particularly suited for the method of making and/or isolating isoidide according to the present invention wherein the reaction is performed in the presence of a metal catalyst, preferably in the presence of a preferred or more preferred metal catalyst as defined here above.

In certain variants of the method of making and/or isolating isoidide according to the present invention as defined here above, step M4) is performed in the presence of one or more organic solvents which are inert (or which are only catalytically active) under the reaction conditions, or step M4) is performed in the absence of such organic solvent or solvents. Suitable inert (or only catalytically active) organic solvents are selected from ether solvents, ester solvents, nitrile solvents, ketone solvents, amide solvents, aromatic solvents, aliphatic solvents, haloaromatic solvents, haloaliphatic solvents and their mixtures, as is generally known in the art. Solvents which are only catalytically active in the reaction are not consumed in the reaction, as is generally known in the art.

In preferred variants of the method of making and/or isolating isoidide according to the present invention, the reagent for reacting with the mixture of compounds of formula II is used as (or is also functioning as) solvent in step M4). In these preferred variants, preferably no additional organic solvents which are inert (or which are only catalytically active, as specified above) under the reaction conditions are present in step M4).

Also preferred is a method of making and/or isolating isoidide according to the present invention as defined herein (or a method of making and/or isolating isoidide according to the present invention as described above or below as being preferred) wherein the mixture of compounds of formula II provided or prepared in step M3) comprises a mixture comprising or consisting of isoidide, isosorbide and isomannide,
wherein preferably
- isoidide is present in a total amount in the range of from 10 to 95 mole-%, preferably in the range of from 40 to 95 mole-%, more preferably in the range of from 50 to 90 mole-%, relative to the total molar amount of the mixture of compounds of formula II,
   or
- isoidide is present in a total amount in the range of from 40 to 70 mole-%, preferably in the range of from 45 to 65 mole-%, more preferably in the range of from 50 to 60 mole-%, relative to the total molar amount of the mixture of compounds of formula II.

Where in the method of making and/or isolating isoidide according to the present invention as defined here above the mixture of compounds of formula II provided or prepared in step M3) comprises a mixture comprising or consisting of isoidide, isosorbide and isomannide, this is preferably prepared from isomerization, respectively from epimerization, in particular under the conditions of a transfer hydrogenation (as explained and specified in more detail below, cf. in particular step M2)), from one or more compounds selected from the group consisting of isosorbide, isomannide, and mixtures thereof, more preferably from isosorbide.

Under the conditions of the method of making and/or isolating isoidide according to the invention as defined herein, preferably under the conditions of step M2), isoidide is usually found to be present in the mixture comprising or consisting of isoidide, isosorbide and isomannide in a total amount in the range of from 40 to 70 mole-%, (or in the preferred amounts as defined above), relative to the total molar amount of the mixture of compounds of formula II, as defined above. Said total amount of isoidide amount in the range of from 40 to 70 mole-% is therefore believed to represent the thermodynamic equilibrium amount of isoidide as resulting from the conditions of the method of making and/or isolating isoidide according to the invention as defined herein, specifically under the conditions of step M2).

Said mixture comprising or consisting of isoidide, isosorbide and isomannide received under the conditions of the method of making and/or isolating isoidide according to the invention as defined herein can, however, be further enriched in isoidide, e.g. by physico-chemical methods generally known in the field, comprising e.g. distillation methods, crystallization methods and chromatographic methods. As a result from these steps of enriching said mixture comprising or consisting of isoidide, isosorbide and isomannide in isoidide, isoidide may be present in a so enriched mixture in a total amount of up to 95 mole-%, as defined above.

Further preferred is a method of making and/or isolating isoidide according to the present invention as defined herein (or a method of making and/or isolating isoidide according to the present invention as described above or below as being preferred) wherein,
- in the mixture comprising non-esterified isoidide and one or both compounds selected from the group consisting of esterified isosorbide and esterified isomannide received in step M4),
- the molar ratio of non-esterified isoidide : esterified isoidide is in the range of from 75 : 25 to 98 : 2, preferably of from 80 : 20 to 95 : 5,
   and/or
- the molar ratio of any present non-esterified isosorbide : any present esterified isosorbide is in the range of from 20 : 80 to 0.01 : 99.99, preferably of from 5 : 95 to 0.5 : 99.5,
   and/or
- the molar ratio of any present non-esterified isomannide : any present esterified isomannide is in the range of from 20 : 80 to 0.01 : 99.99, preferably of from 5 : 95 to 0.1 : 99.9,
   and/or
- the mixture comprising non-esterified isoidide and one or both compounds selected from the group consisting of esterified isosorbide and esterified isomannide received in step M4) comprises isoidide, isomannide-2,5-diacetate and isosorbide monoacetate, preferably isosorbide-5-monoacetate.

It has been found in our own experiments that, depending on the reaction time allowed, the molar ratio of any present esterified (in particular: acetylated) isosorbide : any present non-esterified isosorbide in a mixture comprising non-esterified isoidide and esterified (in particular: mono-acylated, more in particular: mono-acetylated) isosorbide and optionally esterified (in particular: bis-acylated, more in particular: bis-acetylated) isomannide received in step M4) can become ≥ 99 : 1. Receiving such a high molar excess of esterified isosorbide from the method of making and/or isolating isoidide of the present invention is particularly beneficial as it considerably simplifies separation of isosorbide (respectively: esterified isosorbide) from the mixture received in step M4).

Under a further aspect, a method of making and/or isolating isoidide according to the present invention as defined herein (or a method of making and/or isolating isoidide according to the present invention as described above or below as being preferred) is also preferred wherein,
- in the mixture comprising non-esterified isoidide and one or both compounds selected from the group consisting of esterified isosorbide and esterified isomannide received in step M4),
- the molar ratio of non-esterified isoidide : esterified isoidide is ≥ 80 : 20
   and
- the molar ratio of any present non-esterified isosorbide : any present esterified isosorbide is ≤ 5 : 95
   and
- the molar ratio of any present non-esterified isomannide : any present esterified isomannide is ≤ 5 : 95.

Moreover, preferred is a method of making and/or isolating isoidide according to the present invention as defined herein (or a method of making and/or isolating isoidide according to the present invention as described above or below as being preferred), comprising the following additional step (preferably conducted before step M3) as defined above):
M2) reacting one or more compounds selected from the group consisting of isosorbide, isomannide, esters of isosorbide, esters of isomannide and mixtures thereof,
   preferably one or more compounds selected from the group consisting of isosorbide, isomannide, and mixtures thereof, more preferably isosorbide,
   wherein preferably the one or more compounds selected from the group consisting of isosorbide, isomannide, and mixtures thereof, preferably the isosorbide, is (are) used as a mixture of organic (preferably cycloheteroaliphatic) hydroxyl compounds, comprising a total amount of isosorbide in the range of from 90 to 99.5 mass-%, preferably of from 95 to 99.5 mass-%, relative to the total mass of sorbitol, mannitol, isosorbide, isomannide and sorbitan derivatives, wherein the sorbitan derivatives are selected from the group consisting of 1,4-sorbitan, 3,6-sorbitan, 2,5-mannitan, 2,6-sorbitan, 1,5-sorbitan, 2,5 iditan and mixtures thereof, present in the mixture of organic (preferably cycloheteroaliphatic) hydroxyl compounds,
under conditions of transfer hydrogenation in the presence of a transition metal catalyst and preferably in the presence of hydrogen,
   wherein preferably the transition metal is selected from the group consisting of nickel, copper, ruthenium and rhodium, more preferably the transition metal is ruthenium or nickel,
preferably
   - in the presence of a polar solvent, preferably selected from the group consisting of alcohols, preferably alcohols comprising 1 to 6 carbon atoms, water and mixtures thereof,
      more preferably in the presence of a solvent comprising or consisting of one or more alcohols, each comprising 1 to 6, preferably 2 to 4, carbon atoms, even more preferably in the presence of 2-propanol,
      and/or
   - at a total pressure in the range of from 200 to 10000 kPa, preferably of from 1000 to 7500 kPa, more preferably of from 2000 to 5000 kPa,
      and/or
   - at a temperature in the range of from 100 °C to 250 °C, preferably of from 150 °C to 235 °C,
to receive a mixture of compounds of formula II as defined above.

Where in the method of making and/or isolating isoidide according to the present invention as defined here above, step M2) is conducted in an organic and preferably polar solvent as defined above, i.e. in a non-aqueous solvent, this preferred alternative of the method of making and/or isolating isoidide according to the present invention has the beneficial effect that the resulting product from step M2) (i.e. the mixture of compounds of formula II as defined above) can conveniently be used for step M3) and/or step M4) without time-consuming efforts for removing water which might otherwise be necessary, because the reagent for reacting the mixture of compounds of formula II preferably used in step M4) is usually water-sensitive.

Where in the method of making and/or isolating isoidide according to the present invention as defined here above, step M2) is conducted in the presence of a transition metal catalyst, the transition metal catalyst is present in homogenous phase or in heterogeneous phase, preferably in heterogeneous phase. Preferred as transition metal catalyst present in heterogeneous phase in step M2) is a catalyst selected from the group consisting of (i) supported heterogeneous transition metal catalysts, preferably ruthenium on carbon, and (ii) skeletal heterogeneous transition metal catalysts, preferably skeletal heterogeneous nickel catalysts, more preferably skeletal heterogeneous nickel catalysts prepared from a nickel alloy, e.g. as described in US 1,628,190, and generally known in the art as "Raney nickel", even more preferably skeletal heterogeneous nickel catalysts (as defined here before or defined here before as preferred) which are molybdenum-promoted and yet even more preferably molybdenum-promoted Raney nickel catalysts.

Preferably, a transition metal catalyst present in heterogeneous phase in step M2) in the method of making and/or isolating isoidide according to the present invention as defined here above is present in a solid form selected from the group consisting of powder, slurry and formed body (where the formed body preferably comprises extrudates and tablets).

In a preferred variant of the method of making and/or isolating isoidide according to the present invention as defined herein (or a method of making and/or isolating isoidide according to the present invention as described above or below as being preferred), said method comprises the following additional step:
M1) reacting one or both compounds selected from the group consisting of sorbitol (preferably D-sorbitol), mannitol (preferably D-mannitol) and mixtures thereof, under acidic conditions to receive (at least) one or more compounds selected from the group consisting of isosorbide, isomannide and mixtures thereof,
preferably for providing said one or more compounds selected from the group consisting of isosorbide, isomannide and mixtures thereof to be used in (or for) step M2).

The reaction defined in step M1) is generally known in the art *per* se (e.g. at least aspects thereof are known from document US 2009/0259057) as an acid-catalyzed bimolecular dehydration reaction, proceeding via the respective intermediate compounds sorbitans, mannitans or iditans.

In one preferred specific variant of the method of making and/or isolating isoidide according to the present invention as defined herein (or a method of making and/or isolating isoidide according to the present invention as described above or below as being preferred), the one or more compounds selected from the group consisting of isosorbide, isomannide and mixtures thereof which is or which are preferably used in (or for) step M2) (as defined here above) is used as a mixture of organic (preferably cycloheteroaliphatic) hydroxyl compounds, comprising a total amount of isosorbide in the range of from 90 to 99.5 mass-%, preferably of from 95 to 99.5 mass-%, relative to the total mass of sorbitol, mannitol, isosorbide, isomannide and sorbitan derivatives selected from the group consisting of 1,4-sorbitan, 3,6-sorbitan, 2,5-mannitan, 2,6-sorbitan, 1,5-sorbitan, 2,5 iditan and mixtures thereof (as specified below) present in the mixture of organic (preferably cycloheteroaliphatic) hydroxyl compounds. In this preferred specific variant of the method of making and/or isolating isoidide, the mixture of organic (preferably cycloheteroaliphatic) hydroxyl compounds (in addition to isosorbide as defined here before) preferably further comprises a total amount of sorbitan dervatives of ≥ 0.5 mass-%, preferably in the range of from 0.5 mass-% to 5 mass-%, relative to the total mass of sorbitol, mannitol, isosorbide, isomannide and sorbitan derivatives (as defined above) present in the mixture of organic (preferably cycloheteroaliphatic) hydroxyl compounds. Said mixture or mixtures of organic (preferably cycloheteroaliphatic) hydroxyl compounds are known per se in the art (e.g. as "crude isosorbide"), e.g. from C. Dussenne et al., Green Chemistry (2017) 19, 5332-5344. Said sorbitan derivatives as mentioned above which are selected from the group consisting of 1,4-sorbitan, 3,6-sorbitan, 2,5-mannitan, 2,6-sorbitan, 1,5-sorbitan 2,5-iditan and mixtures thereof, have the following chemical structures (as is known in the art):

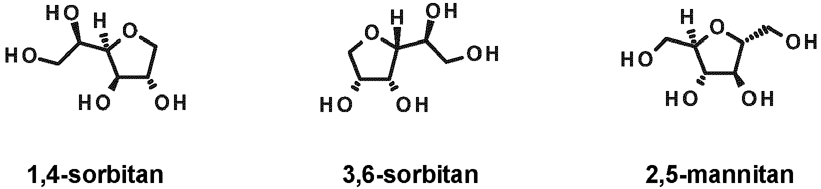

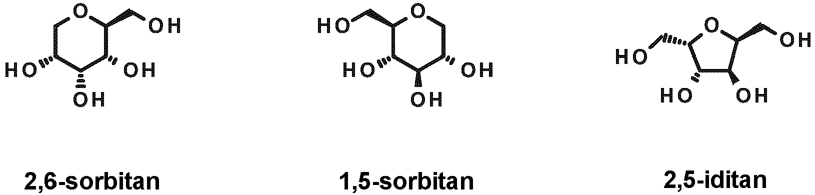

Furthermore, preferred is a method of making and/or isolating isoidide according to the present invention as defined herein (or a method of making and/or isolating isoidide according to the present invention as described above or below as being preferred), further comprising the following additional step:
M5) separating non-esterified isoidide from the mixture comprising non-esterified isoidide and one or both compounds selected from the group consisting of esterified isosorbide and esterified isomannide, received in step M4), preferably by phase separation, more preferably by liquid-liquid extraction,
to obtain and/or isolate non-esterified isoidide.

In the method of making and/or isolating isoidide according to the present invention as defined here above (step M5)), the non-esterified isoidide can be separated from the mixture comprising non-esterified isoidide and one or both compounds selected from the group consisting of esterified isosorbide and esterified isomannide (and optionally also esterified isoidide), received in step M4) preferably by phase separation or by chromatography. Phase separation is a preferred method for separating non-esterified isoidide in step M5) of the method of making and/or isolating isoidide according to the present invention.

Where in the method of making and/or isolating isoidide according to the present invention as defined here above (step M5)) the non-esterified isoidide is separated from the mixture comprising non-esterified isoidide and one or both compounds selected from the group consisting of esterified isosorbide and esterified isomannide (and optionally esterified isoidide), received in step M4), by phase separation, said phase separation preferably comprises liquid-liquid extraction, crystallization and distillation. Liquid-liquid extraction is a preferred method of phase separation of step M5) of the method of the present invention.

It has been found in own experiments that liquid-liquid extraction of an aqueous phase (comprising or receiving at least the majority of the non-esterified isoidide received in step M4)) in step M5) with an organic, water-immiscible solvent, preferably selected from the group consisting of chloroform, 2-methyltetrahydrofuran, isopropyl methyl ketone, ethyl acetate and mixtures thereof, more preferably the organic, water immiscible organic solvent is or comprises ethyl acetate, (comprising or receiving the mixture comprising at least the majority of the one or both compounds selected from the group consisting of esterified isosorbide and esterified isomannide, received in step M4), and optionally also any esterified isoidide) is a particularly mild, convenient and efficient method of separating (by phase separation) non-esterified isoidide in high yield from a mixture comprising non-esterified isoidide and one or both compounds selected from the group consisting of esterified isosorbide and esterified isomannide, and optionally also esterified isoidide.

A method of making and/or isolating isoidide according to the present invention as defined herein (or a method of making and/or isolating isoidide according to the present invention as described above or below as being preferred), is therefore preferred, comprising at least the following steps (i.e. the method of making and/or isolating isoidide according to the present invention may comprise further additional steps, e.g. one or more or all of steps M1), M2), M6) and/or M7), as disclosed herein):
M3) providing or preparing a mixture of compounds of formula II 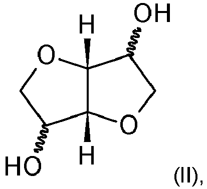
comprising isoidide and one or both compounds selected from the group consisting of isosorbide and isomannide (as explained and specified in more detail above),
M4) subjecting the mixture of compounds of formula II from step M3) to conditions of a selective esterification, so that a mixture comprising non-esterified isoidide and one or both compounds selected from the group consisting of esterified isosorbide and esterified isomannide are (or respectively, is,) received (as explained and specified in more detail above)
wherein the selective esterification is performed in the presence of a metal catalyst and comprises reacting the mixture of compounds of formula II with a reagent selected from the group consisting of carboxylic acid anhydrides wherein the carboxylic acid or carboxylic acids forming the carboxylic acid anhydride in each case are selected from the group consisting of carboxylic acids comprising a total number of carbon atoms in the range of from 2 to 8,
   and
M5) separating non-esterified isoidide from the mixture comprising non-esterified isoidide and one or both compounds selected from the group consisting of esterified isosorbide and esterified isomannide, received in step M4), preferably by phase separation, more preferably by liquid-liquid extraction (as explained and specified in more detail above),
to obtain and/or isolate non-esterified isoidide.

Also preferred is a method of making and/or isolating isoidide according to the present invention as defined herein (or a method of making and/or isolating isoidide according to the present invention as described above or below as being preferred), further comprising the following additional step or steps:
M6) using the mixture comprising one or both compounds selected from the group consisting of esterified isosorbide and esterified isomannide, and preferably further comprising esterified isoidide, received after separation in step M5) and/or one or more reaction products thereof, wherein preferably the one or more reaction products comprise one or more compounds selected from the group consisting of isosorbide, isomannide, and mixtures thereof, and preferably further comprise isoidide, for providing or preparing, preferably in one or more steps, the mixture of compounds of formula II in (or for) step M3);
and/or (preferably "or")
M7) hydrolyzing the mixture comprising one or both compounds selected from the group consisting of esterified isosorbide and esterified isomannide, and preferably further comprising esterified isoidide, received after separation in step M5) and preferably using the so received isosorbide and/or isomannide (and preferably also the so received isoidide) in (or for) step M2).

By steps M6) and/or M7) of the method of making and/or isolating isoidide according to the present invention, any esterified dianhydrohexitol isomers which have not been converted into the desired product isoidide can be recovered and re-cycled into the method or process according to the invention to further increase the overall yield in the desired product isoidide. By performing a method of making and/or isolating isoidide according to the present invention including step M6) and/or M7), the effectiveness of the method can therefore be beneficially further increased.

The present invention also pertains to a composition comprising a mixture comprising (at least) non-esterified isoidide and one or both compounds selected from the group consisting of esterified isosorbide and esterified isomannide, preferably as defined above in (or for) step M4) of the method of making and/or isolating isoidide according to the present invention (or a respective method of making and/or isolating isoidide according to the present invention as described herein as being preferred). Any present esterified isosorbide, any present esterified isomannide and any present esterified isoidide is esterified with a carboxylic acid comprising a total number of carbon atoms in the range of from 2 to 8 and the molar ratio of non-esterified isoidide : esterified isoidide present in the composition is in the range of from 75 : 25 to 98 : 2.

Said composition may comprise further components. For example, said composition may in addition also comprise esterified isoidide and ususally comprises in addition esterified isoidide. The term "esterified isoidide" as used in the context of the present invention comprises the group consisting of monoesters of isoidide, diesters of isoidide and mixtures of monoesters of isoidide and diesters of isoidide, as explained above.

Generally, all aspects of the present invention discussed herein in the context of the method of making and/or isolating isoidide according to the present invention, apply *mutatis mutandis* to the composition comprising a mixture comprising non-esterified isoidide and one or both compounds selected from the group consisting of esterified isosorbide and esterified isomannide according to the present invention, as defined here above and below. And *vice versa,* all aspects of the present invention discussed herein in the context of the composition comprising a mixture comprising non-esterified isoidide and one or both compounds selected from the group consisting of esterified isosorbide and esterified isomannide according to the present invention, apply *mutatis mutandis* to the method of making and/or isolating isoidide according to the present invention as defined herein. Preferred is a composition according to the present invention as defined above (or a composition according to the present invention as described above or below as being preferred) wherein any present esterified isosorbide, any present esterified isomannide and any present esterified isoidide is esterified in each case with a carboxylic acid comprising a total number of carbon atoms in the range of from 2 to 8. Preferably, any present esterified isosorbide, any present esterified isomannide and any present esterified isoidide is esterified in each case with an equal carboxylic acid, comprising in each case a total number of carbon atoms in the range of from 2 to 8. For example, in a so preferred composition according to the present invention, any present esterified isosorbide, any present esterified isomannide and any present esterified isoidide is esterified in each case with acetic acid (i.e. all of said esterified compounds present in the composition are present as acetate esters).

Further preferred is a composition according to the present invention as defined above (or a composition according to the present invention as described above or below as being preferred) wherein
- the esterified isosorbide is esterified by reacting isosorbide with a reagent selected from the group consisting of carboxylic acid anhydrides wherein the carboxylic acid or carboxylic acids forming the carboxylic acid anhydride in each case are selected from the group consisting of carboxylic acids comprising a total number of carbon atoms in the range of from 2 to 8
   and/or
- the esterified isomannide is esterified by reacting isomannide with a reagent selected from the group consisting of carboxylic acid anhydrides wherein the carboxylic acid or carboxylic acids forming the carboxylic acid anhydride in each case are selected from the group consisting of carboxylic acids comprising a total number of carbon atoms in the range of from 2 to 8.

Also preferred is a composition according to the present invention as defined above (or a composition according to the present invention as described above or below as being preferred), wherein
- the molar ratio of non-esterified isoidide present in the composition to the sum of esterified isoidid, esterified isosorbide and esterified isomannide present in the composition is in the range of from 40 : 60 to 65 : 35, preferably of from 45 : 55 to 60 : 40,
   and/or
- the molar ratio of non-esterified isoidide : esterified isoidide present in the composition is in the range of from 75 : 25 to 98 : 2, preferably of from 80 : 20 to 95 : 5,
   and/or
- the molar ratio of any present non-esterified isosorbide : any present esterified isosorbide in the composition is in the range of from 20 : 80 to 0.01 : 99.99, preferably of from 5 : 95 to 0.5 : 99.5,
   and/or
- the molar ratio of any present non-esterified isomannide : any present esterified isomannide in the composition is in the range of from 20 : 80 to 0.01 : 99.99, preferably of from 5 : 95 to 0.1 : 99.9,
   and/or
- the composition comprises isoidide, isomannide-2,5-diacetate and isosorbide monoacetate, preferably isosorbide-5-monoacetate.

In the composition according to the present invention as defined above (or a composition according to the present invention as described above or below as being preferred) wherein
- any present esterified isosorbide, any present esterified isomannide and any present esterified isoidide is esterified in each case with a carboxylic acid comprising a total number of carbon atoms in the range of from 2 to 8
   and
- the molar ratio of non-esterified isoidide : esterified isoidide present in the composition is in the range of from 75 : 25 to 98 : 2, preferably of from 80 : 20 to 95 : 5.

The composition according to the present invention as defined above is a valuable starting material for the method of making and/or isolating isoidide according to the present invention and/or a valuable intermediate in the method of making and/or isolating isoidide according to the present invention.

Also preferred is a composition according to the present invention as defined above (or a composition according to the present invention as described above or below as being preferred), wherein
- the molar ratio of non-esterified isoidide : any present esterified isoidide in the composition is ≥ 80 : 20
   and
- the molar ratio of any present non-esterified isosorbide : any present esterified isosorbide in the composition is ≤ 5 : 95,
   and
- the molar ratio of any present non-esterified isomannide : any present esterified isomannide in the composition is ≤ 5 : 95.

The present invention further pertains to a composition according to the present invention as defined above (or a composition according to the present invention as described above or below as being preferred), obtained or obtainable by a method of making and/or isolating isoidide according to the present invention as defined herein (or by a method of making and/or isolating isoidide according to the present invention as described above or below as being preferred).

Generally, all aspects of the present invention discussed herein in the context of the method of making and/or isolating isoidide according to the present invention as defined above and in the context of the composition comprising a mixture comprising non-esterified isoidide and one or both compounds selected from the group consisting of esterified isosorbide and esterified isomannide according to the present invention, as defined above, apply *mutatis mutandis* to the composition according to the present invention obtainable by a method of making and/or isolating isoidide according to the present invention as defined above and below. And *vice versa,* all aspects of the present invention discussed herein in the context of the composition according to the present invention obtainable by a method of making and/or isolating isoidide according to the present invention as defined above and below apply *mutatis mutandis* to the method of making and/or isolating isoidide according to the present invention as defined above and to the composition comprising a mixture comprising non-esterified isoidide and one or both compounds selected from the group consisting of esterified isosorbide and esterified isomannide according to the present invention, as defined above.

Moreover, the present invention pertains to the use of a composition according to the present invention as defined above (or a composition according to the present invention as described above or below as being preferred),
- in a method for making and/or isolating isoidide, preferably as starting material or as intermediate;
   and/or
- in a method for separating isoidide from other dianhydrohexitols, preferably from a mixture of other dianhydrohexitols or dianhydrohexitol isomers comprising isosorbide and/or isomannide,
preferably as starting material or as intermediate.

Generally, all aspects of the present invention discussed herein in the context of the method of making and/or isolating isoidide according to the present invention as defined above, in the context of the composition comprising a mixture comprising non-esterified isoidide and one or both compounds selected from the group consisting of esterified isosorbide and esterified isomannide according to the present invention, as defined above, and/or in the context of the composition according to the present invention obtainable by a method of making and/or isolating isoidide according to the present invention as defined above apply *mutatis mutandis* to the use of a composition according to the present invention as defined above. And *vice versa,* all aspects of the present invention discussed herein in the context of the use of a composition according to the present invention as defined above apply *mutatis mutandis* to the method of making and/or isolating isoidide according to the present invention as defined above, to the composition comprising a mixture comprising non-esterified isoidide and one or both compounds selected from the group consisting of esterified isosorbide and esterified isomannide according to the present invention, as defined above, and to the composition according to the present invention obtainable by a method of making and/or isolating isoidide according to the present invention as defined above and below.

Furthermore, the present invention pertains to the use of a method comprising a step of selective esterification of a mixture of dianhydrohexitol isomers comprising isoidide (preferably a mixture of compounds of formula II as defined above), preferably a method of making and/or isolating isoidide according to the present invention as defined herein (or a method of making and/or isolating isoidide according to the present invention as described above or below as being preferred), for separating isoidide from said mixture of dianhydrohexitol isomers, preferably from a mixture of compounds of formula II as defined above (or a mixture of compounds of formula II as described above as being preferred).

Generally, all aspects of the present invention discussed herein in the context of the method of making and/or isolating isoidide according to the present invention as defined above, in the context of the composition comprising a mixture comprising non-esterified isoidide and one or both compounds selected from the group consisting of esterified isosorbide and esterified isomannide according to the present invention, as defined above, in the context of the composition according to the present invention obtainable by a method of making and/or isolating isoidide according to the present invention as defined above and/or in the context of the use of a composition as defined above apply *mutatis mutandis* to the use of a method comprising a step of selective esterification according to the present invention as defined above. And *vice versa,* all aspects of the present invention discussed herein in the context of the use of a method comprising a step of selective esterification according to the present invention as defined above apply *mutatis mutandis* to the method of making and/or isolating isoidide according to the present invention as defined above, to the composition comprising a mixture comprising non-esterified isoidide and one or both compounds selected from the group consisting of esterified isosorbide and esterified isomannide according to the present invention, as defined above, to the composition according to the present invention obtainable by a method of making and/or isolating isoidide according to the present invention as defined above and below and to the use of a composition according to the present invention as defined above.

Preferred is the use of a method comprising a step of selective esterification according to the present invention as defined above (or the use of a method comprising a step of selective esterification according to the present invention as described above as being preferred), wherein the method comprises selectively esterifying the dianhydrohexitol isomers otherthan isoidide and subsequently separating the non-esterified isoidide from the mixture of esterified dianhydrohexitol isomers.

As mentioned, isoidide or modified isoidide is of particular interest as a building block (specifically as a monomer) for polymers, e.g. for bio-degradable polymers and/or for polymers derived from renewable natural resources. Examples include polyesters made by polycondensation of isoidide and a dicarboxylic acid or anhydride, and polycarbonates made by reaction with a bifunctional carboxyl compound such as phosgene. Isoidide is also useful in other polymerizations wherein conventionally other diols are used. E.g., bisglycidyl ethers of isoidide can be used as a substitute for bisphenol-A in epoxy resins.

The present invention therefore also pertains to a method of making a polymer comprising isoidide monomers or modified isoidide monomers (and/or to the use of isoidide monomers or modified isoidide monomers for making a polymer) wherein the method (or the use) comprises making or isolating the isoidide monomers and/or the modified isoidide monomers by a method of making and/or isolating isoidide according to the present invention as defined herein (or according to a method of making and/or isolating isoidide according to the present invention as described herein as being preferred), or involving a method of making and/or isolating isoidide according to the present invention as defined herein (or according to a method of making and/or isolating isoidide according to the present invention as described herein as being preferred).

Generally, all aspects of the present invention discussed herein in the context of the method of making and/or isolating isoidide according to the present invention as defined above, in the context of the composition comprising a mixture comprising non-esterified isoidide and one or both compounds selected from the group consisting of esterified isosorbide and esterified isomannide according to the present invention, as defined above, in the context of the composition according to the present invention obtainable by a method of making and/or isolating isoidide according to the present invention as defined above, in the context of the use of a composition as defined above and/or in the context of the use of a method comprising a step of selective esterification according to the present invention as defined above apply *mutatis mutandis* to the method of making (or use for) a polymer as defined above. And *vice versa,* all aspects of the present invention discussed herein in the context of the method of (or use for) making a polymer according to the present invention as defined above apply *mutatis mutandis* to the method of making and/or isolating isoidide according to the present invention as defined above, to the composition comprising a mixture comprising non-esterified isoidide and one or both compounds selected from the group consisting of esterified isosorbide and esterified isomannide according to the present invention, as defined above, to the composition according to the present invention obtainable by a method of making and/or isolating isoidide according to the present invention as defined above and below, to the use of a composition according to the present invention as defined above and to the use of a method comprising a step of selective esterification according to the present invention as defined above.

### Examples:

The following examples according to the present invention are meant to further explain and illustrate the present invention without limiting its scope.

### Example 1: Isomerization of isosorbide (step M2)) using Ru/C as transition metal catalyst

A solution of isosorbide (10.00 g, 68.4 mmole) in isopropanol (20 mL) was stirred in a reactor for 2 hrs with 500 mg of a 5% Ru/C heterogeneous catalyst (5 mass-% Ru/C catalyst, resulting in 0.25 mass-% ruthenium relative to the mass of isosorbide used) under a hydrogen pressure of 10 bar (1000 kPa) and at a temperature of 220 °C ("transfer hydrogenation conditions"). The reactor was then cooled to room temperature and depressurized. The catalyst (Ru/C) was isolated by filtration (whereby > 97% recovery of the catalyst were found) and washed with fresh isopropanol (10 mL). The filtrate (comprising the organic components) was concentrated under reduced pressure to give a colorless, viscous oil, comprising a mixture of dianhydrohexitols which was found (by relative integration of characteristic ¹H-NMR signals of the different dianhydrohexitol components, confirmed by gas chromatography coupled with mass spectrometry, GC-MS) to comprise isoidide (55 mole-% relative to the total molar amount of dianhydrohexitols present in the mixture), isosorbide (38 mole-% relative to the total molar amount of dianhydrohexitols present in the mixture) and isomannide (7 mole-% relative to the total molar amount of dianhydrohexitols present in the mixture).

The colorless, viscous oil received from this reaction was then used for the next reaction step without further purification or isolation of its components.

### Example 2: Selective esterification of a mixture of compounds of formula II (steps M3)) and M4)) - Part I

To the colorless, viscous oil provided as received in example 1 above (comprising isoidide, isosorbide and isomannide; step M3)), acetic anhydride (7.12 mL, 75.3 mmole) and lead(ll) acetate (556 mg, 1.71 mmole) were added and the resulting mixture was stirred at room temperature for 3 hrs. Then, ethyl acetate (30 mL) was added to the mixture, leading to precipitation of the lead (II) acetate catalyst. The precipitated catalyst was subsequently collected by filtration (whereby > 96% recovery of the catalyst were found) and washed with fresh ethyl acetate (5 mL).

The combined ethyl acetate phases (comprising the esterified, specifically: acetylated, mixture of dianhydrohexitols and non-esterified dianhydrohexitols) received from this reaction were then used for the next reaction step without further purification or isolation of its components.

### Example 3: Separation of isoidide from a mixture of dianhydrohexitols by liquid-liquid extraction (step M5)) - Part I

The combined ethyl acetate phases (comprising the esterified, specifically: acetylated, mixture of dianhydrohexitols and non-esterified dianhydrohexitols) received from example 2 above were stirred with water (30 mL) for 30 min, then the layers were separated and the aqueous layer was extracted four times with ethyl acetate (4 x 30 mL), whereby the aqueous phase was stirred again together with the ethyl acetate phase in each case for 30 min before each separation.

The aqueous phase was subsequently separated and the water was removed under reduced pressure to yield isoidide (4.21 g, 42 %, relative to the total amount of isosorbide used as starting material in example 1 above).

The purity of the isoidide obtained in this example 3 was analyzed by gas chromatography coupled with mass spectrometry (GC-MS) and by ¹H-NMR and was found to be ≥ 99%.

### Example 4: Hydrolysis of esterified dianhydrohexitols (step M7))

The organic (ethyl acetate) phases from example 3 above (comprising the esterified, specifically: acetylated, mixture of dianhydrohexitols) were combined and the solvent removed from the combined organic phases under reduced pressure to yield a colorless oil (7.21 g).

The oil so received was dissolved in water (15 mL), acidic cation exchange resin (Amberlite^{®} IR120, 1.0 g) was added and the mixture so received was heated under reflux for 6 hrs. After cooling the mixture to room temperature, the acidic cation exchange resin was collected by filtration (> 99% recovery of the acidic cation exchange resin was found) and the filtrate was concentrated under reduced pressure to yield a mixture of isosorbide, isomannide and isoidide (molar ratio of isomers: 69 (isosorbide) : 13 (isomannide) : 18 (isoidide), combined yield: 5.21 g). The isomeric ratio of the mixture was determined by relative integration of characteristic ¹H-NMR signals of the respective different dianhydrohexitol components.

The mixture of isosorbide, isomannide and isoidide so received in this example 4 was re-cycled for use in a step M2) of the method of making and/or isolating isoidide according to the present invention (see example 5 below).

### Example 5: Recycling a mixture of dianhydrohexitols received in step M7)

The mixture of isosorbide, isomannide and isoidide received in example 4 above was subjected to step M2), using the procedure described in example 1 above, and a mixture comprising isoidide (55 mole-% relative to the total molar amount of dianhydrohexitols present in the mixture), isosorbide (38 mole-% relative to the total molar amount of dianhydrohexitols present in the mixture) and isomannide (7 mole-% relative to the total molar amount of dianhydrohexitols present in the mixture) was obtained.

### Example 6: Isomerization of isosorbide (step M2)) using Raney nickel as transition metal catalyst

A solution of isosorbide (1.00 g, 6.84 mmol) in isopropanol (10 mL) was stirred in a reactor for 3 hrs with a Raney-Nickel catalyst promoted by 1 mass-% Mo (100 mg, "Grace 3202", comprising ≥ 92 % Ni) under a hydrogen pressure of 10 bar (1000 kPa) and at a temperature of 200 °C ("transfer hydrogenation conditions"). The reactor was then cooled to room temperature and depressurized. The catalyst (Raney-Nickel) was isolated by filtration (whereby > 99% recovery of the catalyst were found) and washed with fresh isopropanol (10 mL). The filtrate (comprising the organic components) was concentrated under reduced pressure to give a colorless, viscous oil, comprising a mixture of dianhydrohexitols which was found (by relative integration of characteristic ¹H-NMR signals of the different dianhydrohexitol components and GC-MS) to comprise isoidide (54 mole-% relative to the total molar amount of dianhydrohexitols present in the mixture), isosorbide (39 mole-% relative to the total molar amount of dianhydrohexitols present in the mixture) and isomannide (7 mole-% relative to the total molar amount of dianhydrohexitols present in the mixture).

The colorless, viscous oil received from this reaction was then used for the next reaction step without further purification or isolation of its components.

### Example 7: Selective esterification of a mixture of compounds of formula II (steps M3) and M4)) - Part II

To a colorless, viscous oil provided as received according to the method described above in example 6 (1.0 g, 6.84 mmole; comprising isoidide, isosorbide and isomannide in the molar ratio as shown above in example 6), carboxylic acid anhydride (8.21 mmoles in each case of the carboxylic acid anhydrides as specified for examples 8a to 8 f in table 1 below) and lead (II) acetate trihydrate (65 mg, 0.171 mmole) were added and the resulting mixture was stirred at room temperature for 3 hrs in each case. Then, ethyl acetate (5 mL) was added to the mixture in each case, leading to precipitation of the lead (II) acetate catalyst. The precipitated catalyst was subsequently collected by filtration and washed with fresh ethyl acetate (2 mL).

The combined ethyl acetate phases (comprising the esterified, specifically: acylated, mixture of dianhydrohexitols and non-esterified dianhydrohexitols) received from this reaction were then used in each case for the next reaction step without further purification or isolation of their components.

### Example 8: Separation of isoidide from mixtures of dianhydrohexitols by liquid-liquid extraction (step M5)) - Part II

The combined ethyl acetate phases (comprising the esterified, specifically: acylated, mixture of dianhydrohexitols and non-esterified dianhydrohexitols) received from example 7 above (from several experiments, cf. table 1 below) were evaporated under reduced pressure to receive an oily residue in each case. Each oily residue so received was then dissolved in water (15 mL) and extracted six times with ethyl acetate (6 x 15 mL), using a separatory funnel.

The water was subsequently removed in each case from the aqueous phase under reduced pressure and the organic residue remaining in each case was analyzed by ¹H-NMR and by GC-MS. The respective absolute yields of organic residues (in g) and the results of these analyses of the organic residues are shown in table 1 below for examples 8a to 8f:

**Table 1: Results from the experiments of examples 7 and 8**

| Example No. | Carboxylic acid anhydride | Yield [mass] | Composition (by ¹H-NMR) [mole-%] |
|---|---|---|---|
| 8a | Acetic anhydride | 0.49 g | 90 isoidide : 10 isosorbide monoacetate (2- and 5- isomers) |
| 8b | Propionic anhydride | 0.47 g | 95 isoidide : 5 isosorbide monopropionate (2- and 5- isomers) |
| 8c | Butyric anhydride | 0.44 g | > 99 isoidide |
| 8d | Isobutyric anhydride | 0.48 g | > 99 isoidide |
| 8e | Pivalic anhydride | 0.50 g | > 99 isoidide |
| 8f | Pentanoic anhydride | 0.34 g | > 99 isoidide |

The "Composition" shown in table 1 above refers in each case to the composition of the organic residue obtained, wherein the "mole-%" shown in table 1 for the "Composition" are mole-% of the components of said composition, relative to the total molar amount of dianhydrohexitols (including acylated dianhydrohexitols) present in the organic residue obtained.

From the data shown in table 1 it can i.a. be concluded that at least anhydrides of carboxylic acids with a total number of carbon atoms ≤ 5 allow particularly efficient liquid-liquid extraction with low loss in desired yield, however, at higher cost of reagents if anhydrides of carboxylic acids with a higher total number of carbon atoms are used. Pivalic (acid) anhydride and isobutyric (acid) anhydride (both comprising branched carbon chains) showed the most favorable results in terms of isoidide yield and purity of isoidide so received. It can therefore further be concluded that symmetrical anhydrides of carboxylic acids with a total number of carbon atoms ≤ 5, preferably symmetrical anhydrides of carboxylic acids with a total number of carbon atoms in the range of from ≥ 2 to ≤ 5, wherein preferably the carbon atoms present form (where structurally possible) a branched carbon chain, are particularly preferred as reagents for reacting the mixture of compounds of formula II in step M4) of the method of making and/or isolating isoidide according to the present invention.

## Claims

1. Method of making and/or isolating isoidide of formula I 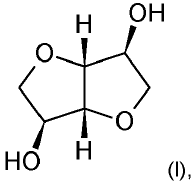 comprising the following steps:
M3) providing or preparing a mixture of compounds of formula II 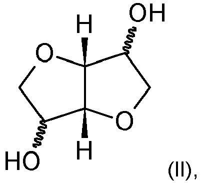 comprising isoidide and one or both compounds selected from the group consisting of isosorbide and isomannide,
M4) subjecting the mixture of compounds of formula II from step M3) to conditions of a selective esterification, so that a mixture comprising non-esterified isoidide and one or both compounds selected from the group consisting of esterified isosorbide and esterified isomannide are received,
wherein the selective esterification
- is performed in the presence of a metal catalyst
and
- comprises reacting the mixture of compounds of formula II with a reagent selected from the group consisting of carboxylic acid anhydrides wherein the carboxylic acid or carboxylic acids forming the carboxylic acid anhydride in each case are selected from the group consisting of carboxylic acids comprising a total number of carbon atoms in the range of from 2 to 8,
and
M5) separating non-esterified isoidide from the mixture comprising non-esterified isoidide and one or both compounds selected from the group consisting of esterified isosorbide and esterified isomannide received in step M4),
to obtain and/or isolate non-esterified isoidide.

2. Method according to claim 1, wherein the selective esterification in step M4)
- is performed in a way to selectively esterify isosorbide and/or isomannide in the presence of isoidide;
and/or
- is performed in the presence of a metal catalyst,
wherein the metal is selected from the group consisting of calcium, strontium, barium, zinc, cadmium, mercury, indium, thallium, the lanthanides, tin, lead, antimony, bismuth, iron, cobalt and nickel;
preferably in the presence of a metal salt catalyst,
wherein preferably the metal is selected from the group consisting of calcium, strontium, barium, zinc, cadmium, mercury, indium, thallium, the lanthanides, tin, lead, antimony, bismuth, iron, cobalt and nickel
and/or
- is performed at a temperature in the range of from -20 °C to 50 °C, preferably of from 10 °C to 50 °C, more preferably of from 15 °C to 45 °C, even more preferably of from 15 °C to 35 °C;
and/or
- is performed for a reaction time in the range of from 15 min to 24 hrs, preferably of from 30 min to 10 hrs, more preferably of from 1 h to 5 hrs, yet more preferably of from 1 h to 3 hrs.

3. Method according to any of the preceding claims, wherein the selective esterification in step M4) is performed in the presence of a metal catalyst, wherein the metal catalyst is or comprises a lead catalyst, preferably a lead salt catalyst.

4. Method according to any of the preceding claims, wherein the mixture of compounds of formula II provided or prepared in step M3) comprises a mixture comprising or consisting of isoidide, isosorbide and isomannide,
wherein preferably
- isoidide is present in a total amount in the range of from 10 to 95 mole-%, preferably in the range of from 40 to 95 mole-%, more preferably in the range of from 50 to 90 mole-%, relative to the total molar amount of the mixture of compounds of formula II,
or
- isoidide is present in a total amount in the range of from 40 to 70 mole-%, preferably in the range of from 45 to 65 mole-%, more preferably in the range of from 50 to 60 mole-%, relative to the total molar amount of the mixture of compounds of formula II.

5. Method according to any of the preceding claims wherein
- in the mixture comprising non-esterified isoidide and one or both compounds selected from the group consisting of esterified isosorbide and esterified isomannide received in step M4),
- the molar ratio of non-esterified isoidide : esterified isoidide is in the range of from 75 : 25 to 98 : 2, preferably of from 80 : 20 to 95 : 5,
and/or
- the molar ratio of any present non-esterified isosorbide : any present esterified isosorbide is in the range of from 20 : 80 to 0.01 : 99.99, preferably of from 5 : 95 to 0.5 : 99.5,
and/or
- the molar ratio of any present non-esterified isomannide : any present esterified isomannide is in the range of from 20 : 80 to 0.01 : 99.99, preferably of from 5 : 95 to 0.1 : 99.9,
and/or
- the mixture comprising non-esterified isoidide and one or both compounds selected from the group consisting of esterified isosorbide and esterified isomannide received in step M4) comprises isoidide, isomannide-2,5-diacetate and isosorbide monoacetate, preferably isosorbide-5-monoacetate.

6. Method according to any of the preceding claims, preferably according to claim 4, comprising the following additional step:
M2) reacting one or more compounds selected from the group consisting of isosorbide, isomannide, esters of isosorbide, esters of isomannide and mixtures thereof
preferably one or more compounds selected from the group consisting of isosorbide, isomannide, and mixtures thereof, more preferably isosorbide,
wherein preferably the one or more compounds selected from the group consisting of isosorbide, isomannide and mixtures thereof, is used as a mixture of organic hydroxyl compounds, comprising a total amount of isosorbide in the range of from 90 to 99.5 mass-%, preferably of from 95 to 99.5 mass-%, relative to the total mass of sorbitol, mannitol, isosorbide, isomannide and sorbitan derivatives, wherein the sorbitan derivatives are selected from the group consisting of 1 ,4-sorbitan, 3,6-sorbitan, 2,5-mannitan, 2,6-sorbitan, 1 ,5-sorbitan, 2,5 iditan and mixtures thereof, present in the mixture of organic hydroxyl compounds,
under conditions of transfer hydrogenation in the presence of a transition metal catalyst and preferably in the presence of hydrogen,
preferably in the presence of a polar solvent, preferably selected from the group consisting of alcohols, water and mixtures thereof,
to receive a mixture of compounds of formula II as defined in any of claims 1 or 4.

7. Method according to claim 6, comprising the following additional step:
M1) reacting one or both compounds selected from the group consisting of sorbitol, mannitol and mixtures thereof, under acidic conditions, to receive one or more compounds selected from the group consisting of isosorbide, isomannide and mixtures thereof,
preferably for providing said one or more compounds selected from the group consisting of isosorbide, isomannide, and mixtures thereof to be used in step M2),

8. Method according to any of the preceding claims, wherein step M5) comprises:
M5) separating non-esterified isoidide from the mixture comprising non-esterified isoidide and one or both compounds selected from the group consisting of esterified isosorbide and esterified isomannide received in step M4) by phase separation, preferably by liquid-liquid extraction,
to obtain and/or isolate non-esterified isoidide.

9. Method according to any of the preceding claims, further comprising the following additional step or steps:
M6) using the mixture comprising one or both compounds selected from the group consisting of esterified isosorbide and esterified isomannide, and preferably further comprising esterified isoidide, received after separation in step M5) and/or one or more reaction products thereof, wherein preferably the one or more reaction products comprise one or more compounds selected from the group consisting of isosorbide, isomannide, and mixtures thereof, and preferably further comprise isoidide,
for providing or preparing, preferably in one or more steps, the mixture of compounds of formula II in step M3);
and/or
M7) hydrolyzing the mixture comprising one or both compounds selected from the group consisting of esterified isosorbide and esterified isomannide, and preferably further comprising esterified isoidide, received after separation in step M5)
and preferably using the so received isosorbide and/or isomannide in step M2).

10. Composition comprising a mixture comprising non-esterified isoidide and one or both compounds selected from the group consisting of esterified isosorbide and esterified isomannide,
wherein
any present esterified isosorbide, any present esterified isomannide and any present esterified isoidide is esterified with a carboxylic acid comprising a total number of carbon atoms in the range of from 2 to 8
and
the molar ratio of non-esterified isoidide : esterified isoidide present in the composition is in the range of from 75 : 25 to 98 : 2.

11. Composition according to claim 10, wherein
- the molar ratio of non-esterified isoidide present in the composition to the sum of esterified isoidide, esterified isosorbide and esterified isomannide present in the composition is in the range of from 40 : 60 to 65 : 35, preferably of from 45 : 55 to 60 : 40,
and/or
- the molar ratio of non-esterified isoidide : esterified isoidide present in the composition is in the range of from 80 : 20 to 95 : 5,
and/or
- the molar ratio of any present non-esterified isosorbide : any present esterified isosorbide in the composition is in the range of from 20 : 80 to 0.01 : 99.99, preferably of from 5 : 95 to 0.5 : 99.5,
and/or
- the molar ratio of any present non-esterified isomannide : any present esterified isomannide in the composition is in the range of from 20 : 80 to 0.01 : 99.99, preferably of from 5 : 95 to 0.1 : 99.9,
and/or
- the composition comprises isoidide, isomannide-2,5-diacetate and isosorbide monoacetate, preferably isosorbide-5-monoacetate.

12. Composition as defined in any of claims 10 to 11, obtained or obtainable by a method according to any of claims 1 to 9.

13. Use of a composition as defined in any of claims 10 to 12
- in a method for making and/or isolating isoidide, preferably as starting material or as intermediate;
and/or
- in a method for separating isoidide from other dianhydrohexitols, preferably from a mixture of other dianhydrohexitols or dianhydrohexitol isomers comprising isosorbide and/or isomannide,
preferably as starting material or as intermediate.

14. Use of a method according to any of claims 1 to 9 comprising a step of selective esterification of a mixture of dianhydrohexitol isomers comprising isoidide, for separating isoidide from said mixture of dianhydrohexitol isomers, preferably from a mixture of compounds of formula II as defined in claim 1 or 4,
wherein preferably the method comprises selectively esterifying the dianhydrohexitol isomers other than isoidide and subsequently separating the non-esterified isoidide from the mixture of esterified dianhydrohexitol isomers.

15. Method of making a polymer comprising isoidide monomers or modified isoidide monomers, wherein the method comprises making or isolating the isoidide monomers and/or the modified isoidide monomers by a method according to any of claims 1 to 9 or involving a method according to any of claims 1 to 9.

## Patentansprüche

1. Verfahren zur Herstellung und/oder Isolierung von Isoidid der Formel I 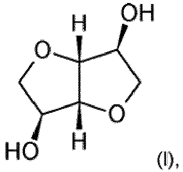 bei dem man:
M3) ein Gemisch von Verbindungen der Formel II 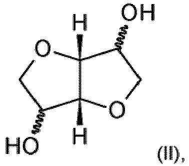 das Isoidid und eine oder beide Verbindungen aus der Gruppe bestehend aus Isosorbid und Isomannid umfasst, bereitstellt und/oder herstellt,
M4) das Gemisch von Verbindungen der Formel II aus Schritt M3) Bedingungen einer selektiven Veresterung unterwirft, so dass ein Gemisch, das nicht verestertes Isoidid und eine oder beide Verbindungen aus der Gruppe bestehend aus verestertem Isosorbid und verestertem Isomannid umfasst, erhalten wird,
wobei die selektive Veresterung
- in Gegenwart eines Metallkatalysators
durchgeführt wird
und
- die Umsetzung des Gemischs von Verbindungen der Formel II mit einem Reagenz aus der Gruppe bestehend aus Carbonsäureanhydriden umfasst, wobei die Carbonsäure bzw. die Carbonsäuren, das bzw. die das Carbonsäureanhydrid bilden, jeweils aus der Gruppe bestehend aus Carbonsäuren mit einer Gesamtzahl von Kohlenstoffatomen im Bereich von 2 bis 8 ausgewählt werden,
und
M5) das nicht veresterte Isoidids aus dem in Schritt M4) erhaltenen Gemisch, das nicht verestertes Isoidid und eine oder beide Verbindungen aus der Gruppe bestehend aus verestertem Isosorbid und verestertem Isomannid umfasst, abtrennt,
unter Erhalt und/oder Isolierung von nicht verestertem Isoidid.

2. Verfahren nach Anspruch 1, wobei die selektive Veresterung in Schritt M4)
- so durchgeführt wird, dass Isosorbid und/oder Isomannid in Gegenwart von Isoidid selektiv verestert wird bzw. werden;
und/oder
- in Gegenwart eines Metallkatalysators durchgeführt wird,
wobei das Metall aus der Gruppe bestehend aus Calcium, Strontium, Barium, Zink, Cadmium, Quecksilber, Indium, Thallium, den Lanthaniden, Zinn, Blei, Antimon, Bismut, Eisen, Cobalt und Nickel ausgewählt ist;
vorzugsweise in Gegenwart eines Metallsalzkatalysators,
wobei das Metall vorzugsweise aus Calcium, Strontium, Barium, Zink, Cadmium, Quecksilber, Indium, Thallium, den Lanthaniden, Zinn, Blei, Antimon, Bismut, Eisen, Cobalt und Nickel ausgewählt ist;
und/oder
- bei einer Temperatur im Bereich von -20 °C bis 50 °C, vorzugsweise von 10 °C bis 50 °C, weiter bevorzugt von 15 °C bis 45 °C, noch weiter bevorzugt von 15 °C bis 35 °C, durchgeführt wird;
und/oder
- über eine Reaktionszeit im Bereich von 15 min bis 24 h, vorzugsweise von 30 min bis 10 h, weiter bevorzugt von 1 h bis 5 h, noch weiter bevorzugt von 1 h bis 3 h, durchgeführt wird.

3. Verfahren nach einem der vorhergehenden Ansprüche, wobei die selektive Veresterung in Schritt M4) in Gegenwart eines Metallkatalysators durchgeführt wird, wobei der Metallkatalysator ein Bleikatalysator, vorzugsweise ein Bleisalzkatalysator, ist oder einen solchen Katalysator umfasst.

4. Verfahren nach einem der vorhergehenden Ansprüche, wobei das in Schritt M3) bereitgestellte oder hergestellte Gemisch von Verbindungen der Formel II ein Gemisch umfasst, das Isoidid, Isosorbid und Isomannid umfasst oder daraus besteht,
wobei vorzugsweise
- Isoidid in einer Gesamtmenge im Bereich von 10 bis 95 Mol-%, vorzugsweise im Bereich von 40 bis 95 Mol-%, weiter bevorzugt im Bereich von 50 bis 90 Mol-%, bezogen auf die gesamte molare Menge des Gemischs von Verbindungen der Formel II, vorliegt
oder
- Isoidid in einer Gesamtmenge im Bereich von 40 bis 70 Mol-%, vorzugsweise im Bereich von 45 bis 65 Mol-%, weiter bevorzugt im Bereich von 50 bis 60 Mol-%, bezogen auf die gesamte molare Menge des Gemischs von Verbindungen der Formel II, vorliegt.

5. Verfahren nach einem der vorhergehenden Ansprüche, wobei
- in dem in Schritt M4) erhaltenen Gemisch, das nicht verestertes Isoidid und eine oder beide Verbindungen aus der Gruppe bestehend aus verestertem Isosorbid und verestertem Isomannid umfasst,
- das Molverhältnis von nicht verestertem Isoidid : verestertem Isoidid im Bereich von 75 : 25 bis 98 : 2, vorzugsweise von 80 : 20 bis 95 : 5, liegt
und/oder
- das Molverhältnis von jeglichem vorliegenden nicht verestertem Isosorbid : jeglichem vorliegenden verestertem Isosorbid im Bereich von 20 : 80 bis 0,01 : 99,99, vorzugsweise von 5 : 95 bis 0,5 : 99,5, liegt
und/oder
- das Molverhältnis von jeglichem vorliegenden nicht verestertem Isomannid : jeglichem vorliegenden verestertem Isomannid im Bereich von 20 : 80 bis 0,01 : 99,99, vorzugsweise von 5 : 95 bis 0,1 : 99,9, liegt
und/oder
- das in Schritt M4) erhaltene Gemisch, das nicht verestertes Isoidid und eine oder beide Verbindungen aus der Gruppe bestehend aus verestertem Isosorbid und verestertem Isomannid umfasst, losidid, Isommanid-2,5-diacetat und Isosorbidmonoacetat, vorzugsweise Isosorbid-5-monoacetat, umfasst.

6. Verfahren nach einem der vorhergehenden Ansprüche, vorzugsweise nach Anspruch 4, das den folgenden zusätzlichen Schritt umfasst:
M2) Umsetzen von einer oder mehreren Verbindungen aus der Gruppe bestehend aus Isosorbid, Isomannid, Estern von Isosorbid, Estern von Isomannid und Gemischen davon,
vorzugsweise einer oder mehreren Verbindungen aus der Gruppe bestehend aus Isosorbid, Isomannid und Gemischen davon, weiter bevorzugt Isosorbid,
wobei vorzugsweise die eine oder die mehreren Verbindungen aus der Gruppe bestehend aus Isosorbid, Isomannid und Gemischen davon als Gemisch von organischen Hydroxylverbindungen verwendet wird, das eine Gesamtmenge von Isosorbid im Bereich von 90 bis 99,5 Massen-%, vorzugsweise von 95 bis 99,5 Massen-%, bezogen auf die Gesamtmasse von Sorbitol, Mannitol, Isosorbid, Isomannid und Sorbitanderivaten, wobei die Sorbitanderivate aus der Gruppe bestehend aus 1,4-Sorbitan, 3,6-Sorbitan, 2,5-Mannitan, 2,6-Sorbitan, 1,5-Sorbitan, 2,5-Iditan und Gemischen davon ausgewählt sind, die in dem Gemisch von organischen Hydroxylverbindungen vorliegen, umfasst, unter Transferhydrierungsbedingungen in Gegenwart eines Übergangsmetallkatalysators und vorzugsweise in Gegenwart von Wasserstoff,
vorzugsweise in Gegenwart eines polaren Lösungsmittels, das vorzugsweise aus der Gruppe bestehend aus Alkoholen, Wasser und Gemischen davon ausgewählt wird, unter Erhalt eines Gemischs von Verbindungen der Formel II gemäß einem der Ansprüche 1 oder 4.

7. Verfahren nach Anspruch 6, das den folgenden zusätzlichen Schritt umfasst:
M1) Umsetzen einer oder beider Verbindungen aus der Gruppe bestehend aus Sorbitol, Mannitol und Gemischen davon unter sauren Bedingungen unter Erhalt einer oder mehrerer Verbindungen aus der Gruppe bestehend aus Isosorbid, Isomannid und Gemischen davon,
vorzugsweise zur Bereitstellung einer oder mehrerer Verbindungen aus der Gruppe bestehend aus Isosorbid, Isomannid und Gemischen davon zur Verwendung in Schritt M2).

8. Verfahren nach einem der vorhergehenden Ansprüche, wobei Schritt M5) Folgendes umfasst:
M5) Abtrennen des nicht veresterten Isoidids aus dem in Schritt M4) erhaltenen Gemisch, das nicht verestertes Isoidid und eine oder beide Verbindungen aus der Gruppe bestehend aus verestertem Isosorbid und verestertem Isomannid umfasst, durch Phasentrennung, vorzugsweise durch Flüssig-Flüssig-Extraktion
unter Erhalt und/oder Isolierung von nicht verestertem Isoidid.

9. Verfahren nach einem der vorhergehenden Ansprüche, ferner umfassend den folgenden zusätzlichen Schritt bzw. die folgenden zusätzlichen Schritte:
M6) Verwenden des nach der Abtrennung in Schritt M5) erhaltenen Gemischs, das eine oder beide Verbindungen aus der Gruppe bestehend aus verestertem Isosorbid und verestertem Isomannid umfasst und vorzugsweise ferner verestertes Isoidid umfasst, und/oder eines oder mehrerer Reaktionsprodukte davon, wobei das eine oder die mehreren Reaktionsprodukte vorzugsweise eine oder mehrere Verbindungen aus der Gruppe bestehend aus Isosorbid, Isomannid und Gemischen davon umfassen und vorzugsweise ferner Isoidid umfassen, zum Bereitstellen oder Herstellen des Gemischs von Verbindungen der Formel II in Schritt M3), vorzugsweise in einem oder mehreren Schritten;
und/oder
M7) Hydrolysieren des nach der Abtrennung in Schritt M5) erhaltenen Gemischs, das eine oder beide Verbindungen aus der Gruppe bestehend aus verestertem Isosorbid und verestertem Isomannid umfasst und vorzugsweise ferner verestertes Isoidid umfasst, und vorzugsweise Verwenden des so erhaltenen Isosorbids und/oder Isomannids in Schritt M2).

10. Zusammensetzung, umfassend ein Gemisch, umfassend nicht verestertes Isoidid und eine oder beide Verbindungen aus der Gruppe bestehend aus verestertem Isosorbid und verestertem Isomannid, wobei
jegliches vorliegende veresterte Isosorbid, jegliches vorliegende veresterte Isomannid und jegliches vorliegende veresterte Isoidid mit einer eine Gesamtzahl von Kohlenstoffatomen im Bereich von 2 bis 8 umfassenden Carbonsäure verestert ist
und
das Molverhältnis von nicht verestertem Isoidid : verestertem Isoidid in der Zusammensetzung im Bereich von 75 : 25 bis 98 : 2 liegt.

11. Zusammensetzung nach Anspruch 10, wobei
- das Molverhältnis von nicht verestertem Isoidid, das in der Zusammensetzung vorliegt, zur Summe von verestertem Isoidid, verestertem Isosorbid und verestertem Isomannid, die in der Zusammensetzung vorliegt, im Bereich von 40 : 60 bis 65 : 35, vorzugsweise von 45 : 55 bis 60 : 40, liegt
und/oder
- das Molverhältnis von nicht verestertem Isoidid : verestertem Isoidid in der Zusammensetzung im Bereich von 80 : 20 bis 95 : 5 liegt
und/oder
- das Molverhältnis von jeglichem vorliegenden nicht veresterten Isosorbid : jeglichem vorliegenden veresterten Isosorbid in der Zusammensetzung im Bereich von 20 : 80 bis 0,01 : 99,99, vorzugsweise von 5 : 95 bis 0,5 : 99,5, liegt
und/oder
- das Molverhältnis von jeglichem vorliegenden nicht veresterten Isomannid : jeglichem vorliegenden veresterten Isomannid in der Zusammensetzung im Bereich von 20 : 80 bis 0,01 : 99,99, vorzugsweise von 5 : 95 bis 0,1 : 99,9, liegt
und/oder
- die Zusammensetzung Iosidid, Isomannid-2,5-diacetat und Isosorbidmonoacetat, vorzugsweise Isosorbid-5-monoacetat, umfasst.

12. Zusammensetzung gemäß einem der Ansprüche 10 bis 11, erhalten oder erhältlich durch ein Verfahren nach einem der Ansprüche 1 bis 9.

13. Verwendung einer Zusammensetzung gemäß einem der Ansprüche 10 bis 12
- bei einem Verfahren zum Herstellen und/oder Isolieren von Isoidid, vorzugsweise als Ausgangsstoff oder als Zwischenprodukt;
und/oder
- bei einem Verfahren zum Abtrennen von Isoidid von anderen Dianhydrohexitolen, vorzugsweise von einem Gemisch von anderen Dianhydrohexitolen oder Dianhydrohexitol-Isomeren, das Isosorbid und/oder Isomannid umfasst,
vorzugsweise als Ausgangsstoff oder als Zwischenprodukt.

14. Verwendung des Verfahrens nach einem der Ansprüche 1 bis 9, umfassend einen Schritt der selektiven Veresterung eines Gemischs von Dianhydrohexitol-Isomeren, das Isoidid umfasst, zum Abtrennen von Isoidid aus dem Gemisch von Dianhydrohexitol-Isomeren, vorzugsweise aus einem Gemisch von Verbindungen der Formel II gemäß Anspruch 1 oder 4, wobei das Verfahren vorzugsweise das selektive Verestern der Dianhydrohexitol-Isomere, die von Isoidid verschieden sind, und das anschließende Abtrennen des nicht veresterten Isoidids aus dem Gemisch von veresterten Dianhydrohexitol-Isomeren umfasst.

15. Verfahren zur Herstellung eines Polymers, das Isoidid-Monomere oder modifizierte Isoidid-Monomere umfasst, wobei das Verfahren das Herstellen oder Isolieren der Isoidid-Monomere oder modifizierten Isoidid-Monomere durch ein Verfahren nach einem der Ansprüche 1 bis 9 oder unter Einbeziehung eines Verfahrens nach einem der Ansprüche 1 bis 9 umfasst.

## Revendications

1. Procédé de fabrication et/ou d'isolement d'iso-idide de formule I 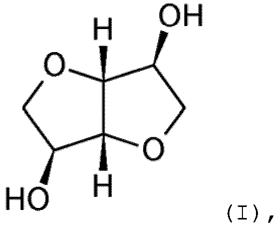 comprenant les étapes suivantes :
M3) fourniture ou préparation d'un mélange de composés de formule II 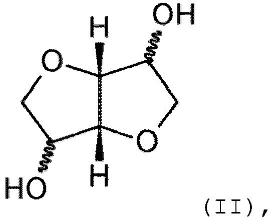
comprenant de l'iso-idide et l'un ou les deux composés choisis dans le groupe constitué de l'isosorbide et de l'isomannide,
M4) soumission du mélange de composés de formule II provenant de l'étape M3) à des conditions d'estérification sélective, de sorte qu'un mélange comprenant de l'iso-idide non estérifié et l'un ou les deux composés choisis dans le groupe constitué de l'isosorbide estérifié et de l'isomannide estérifié sont reçus,
dans lequel l'estérification sélective
- est effectuée en présence d'un catalyseur métallique et
- comprend la réaction du mélange de composés de formule II avec un réactif choisi dans le groupe constitué d'anhydrides d'acide carboxylique dans lesquels l'acide carboxylique ou les acides carboxyliques formant l'anhydride d'acide carboxylique sont dans chaque cas choisis dans le groupe constitué d'acides carboxyliques comprenant un nombre total d'atomes de carbone dans la plage de 2 à 8,
et
M5) séparation de l'iso-idide non estérifié du mélange comprenant de l'iso-idide non estérifié et l'un ou les deux composés choisis dans le groupe constitué de l'isosorbide estérifié et de l'isomannide estérifié reçus dans l'étape M4),
pour obtenir et/ou isoler l'iso-idide non estérifié.

2. Procédé selon la revendication 1, dans lequel l'estérification sélective dans l'étape M4)
- est effectuée de façon à estérifier sélectivement l'isosorbide et/ou l'isomannide en présence d'iso-idide ;
et/ou
- est effectuée en présence d'un catalyseur métallique,
dans lequel le métal est choisi dans le groupe constitué du calcium, du strontium, du baryum, du zinc, du cadmium, du mercure, de l'indium, du thallium, des lanthanides, de l'étain, du plomb, de l'antimoine, du bismuth, du fer, du cobalt et du nickel ;
de préférence en présence d'un catalyseur à base de sel métallique,
dans lequel, de préférence, le métal est choisi dans le groupe constitué du calcium, du strontium, du baryum, du zinc, du cadmium, du mercure, de l'indium, du thallium, des lanthanides, de l'étain, du plomb, de l'antimoine, du bismuth, du fer, du cobalt et du nickel et/ou
- est effectuée à une température dans la plage de -20 °C à 50 °C, de préférence de 10 °C à 50 °C, plus préférablement de 15 °C à 45 °C, encore plus préférablement de 15 °C à 35 °C ;
et/ou
- est effectuée pendant une durée de réaction dans la plage de 15 min à 24 h, de préférence de 30 min à 10 h, plus préférablement de 1 h à 5 h, encore plus préférablement de 1 h à 3 h.

3. Procédé selon l'une quelconque des revendications précédentes, dans lequel l'estérification sélective dans l'étape M4) est effectuée en présence d'un catalyseur métallique, dans lequel le catalyseur métallique est ou comprend un catalyseur au plomb, de préférence un catalyseur à base de sel de plomb.

4. Procédé selon l'une quelconque des revendications précédentes, dans lequel le mélange de composés de formule II fourni ou préparé dans l'étape M3) comprend un mélange comprenant ou constitué de l'iso-idide, de l'isosorbide et de l'isomannide,
dans lequel, de préférence
- l'iso-idide est présent en une quantité totale dans la plage de 10 à 95 % en moles, de préférence dans la plage de 40 à 95 % en moles, plus préférablement dans la plage de 50 à 90 % en moles, par rapport à la quantité molaire totale du mélange de composés de formule II,
ou
- l'iso-idide est présent en une quantité totale dans la plage de 40 à 70 % en moles, de préférence dans la plage de 45 à 65 % en moles, plus préférablement dans la plage de 50 à 60 % en moles, par rapport à la quantité molaire totale du mélange de composés de formule II.

5. Procédé selon l'une quelconque des revendications précédentes dans lequel
- dans le mélange comprenant de l'iso-idide non estérifié et l'un ou les deux composés choisis dans le groupe constitué de l'isosorbide estérifié et de l'isomannide estérifié reçus dans l'étape M4),
- le rapport molaire iso-idide non estérifié : iso-idide estérifié est dans la plage de 75:25 à 98:2, de préférence de 80:20 à 95:5,
et/ou
- le rapport molaire isosorbide non estérifié éventuellement présent : isosorbide estérifié éventuellement présent est dans la plage de 20:80 à 0,01:99,99, de préférence de 5:95 à 0,5:99,5,
et/ou
- le rapport molaire isomannide non estérifié éventuellement présent : isomannide estérifié éventuellement présent est dans la plage de 20:80 à 0,01:99,99, de préférence de 5:95 à 0,1:99,9,
et/ou
- le mélange comprenant de l'iso-idide non estérifié et l'un ou les deux composés choisis dans le groupe constitué de l'isosorbide estérifié et de l'isomannide estérifié reçu dans l'étape M4) comprend de l'iso-idide, du 2,5-diacétate d'isomannide et du monoacétate d'isosorbide, de préférence le 5-monoacétate d'isosorbide.

6. Procédé selon l'une quelconque des revendications précédentes, de préférence selon la revendication 4, comprenant l'étape supplémentaire suivante :
M2) réaction d'un ou plusieurs composés choisis dans le groupe constitué de l'isosorbide, de l'isomannide, d'esters d'isosorbide, d'esters d'isomannide et des mélanges de ceux-ci
de préférence un ou plusieurs composés choisis dans le groupe constitué de l'isosorbide, de l'isomannide, et des mélanges de ceux-ci, plus préférablement l'isosorbide,
dans lequel, de préférence, les un ou plusieurs composés choisis dans le groupe constitué de l'isosorbide, de l'isomannide et des mélanges de ceux-ci, sont utilisés sous la forme d'un mélange de composés hydroxylés organiques, comprenant une quantité totale d'isosorbide dans la plage de 90 à 99,5 % en masse, de préférence de 95 à 99,5 % en masse, par rapport à la masse totale de sorbitol, de mannitol, d'isosorbide, d'isomannide et de dérivés de sorbitane, dans lequel les dérivés de sorbitane sont choisis dans le groupe constitué de : 1,4-sorbitane, 3,6-sorbitane, 2,5-mannitane, 2,6-sorbitane, 1,5-sorbitane, 2,5-iditane et des mélanges de ceux-ci, présents dans le mélange de composés hydroxylés organiques,
dans des conditions d'hydrogénation de transfert en présence d'un catalyseur à métal de transition et de préférence en présence d'hydrogène,
de préférence en présence d'un solvant polaire, de préférence choisi dans le groupe constitué d'alcools, de l'eau et des mélanges de ceux-ci,
pour recevoir un mélange de composés de formule II tel que défini dans l'une quelconque des revendications 1 ou 4.

7. Procédé selon la revendication 6, comprenant l'étape supplémentaire suivante :
M1) réaction de l'un ou des deux composés choisis dans le groupe constitué du sorbitol, du mannitol et des mélanges de ceux-ci, dans des conditions acides, pour recevoir un ou plusieurs composés choisis dans le groupe constitué de l'isosorbide, de l'isomannide et des mélanges de ceux-ci,
de préférence pour fournir lesdits un ou plusieurs composés choisis dans le groupe constitué de l'isosorbide, de l'isomannide, et des mélanges de ceux-ci devant être utilisés dans l'étape M2),

8. Procédé selon l'une quelconque des revendications précédentes, dans lequel l'étape M5) comprend :
M5) la séparation de l'iso-idide non estérifié du mélange comprenant de l'iso-idide non estérifié et l'un ou les deux composés choisis dans le groupe constitué de l'isosorbide estérifié et de l'isomannide estérifié reçus dans l'étape M4) par séparation de phase, de préférence par extraction liquide-liquide,
pour obtenir et/ou isoler l'iso-idide non estérifié.

9. Procédé selon l'une quelconque des revendications précédentes, comprenant en outre l'étape ou les étapes supplémentaires suivantes :
M6) utilisation du mélange comprenant l'un ou les deux composés choisis dans le groupe constitué de l'isosorbide estérifié et de l'isomannide estérifié, et de préférence comprenant en outre de l'iso-idide estérifié, reçu après la séparation dans l'étape M5) et/ou un ou plusieurs produits de réaction de ceux-ci, dans lequel, de préférence, les un ou plusieurs produits de réaction comprennent un ou plusieurs composés choisis dans le groupe constitué de l'isosorbide, de l'isomannide, et des mélanges de ceux-ci, et de préférence, comprennent en outre l'iso-idide,
pour fournir ou préparer, de préférence en une ou plusieurs étapes, le mélange de composés de formule II dans l'étape M3) ;
et/ou
M7) hydrolyse du mélange comprenant l'un ou les deux composés choisis dans le groupe constitué de l'isosorbide estérifié et de l'isomannide estérifié, et de préférence comprenant en outre de l'iso-idide estérifié, reçu après la séparation dans l'étape M5)
et de préférence, utilisation de l'isosorbide et/ou de l'isomannide reçus ainsi dans l'étape M2).

10. Composition comprenant un mélange comprenant de l'iso-idide non estérifié et l'un ou les deux composés choisis dans le groupe constitué de l'isosorbide estérifié et de l'isomannide estérifié,
dans laquelle
l'isosorbide estérifié éventuellement présent, l'isomannide estérifié éventuellement présent et l'iso-idide estérifié éventuellement présent sont estérifiés avec un acide carboxylique comprenant un nombre total d'atomes de carbone dans la plage de 2 à 8
et
le rapport molaire iso-idide non estérifié : iso-idide estérifié présent dans la composition est dans la plage de 75:25 à 98:2.

11. Composition selon la revendication 10, dans laquelle
- le rapport molaire de l'iso-idide non estérifié présent dans la composition à la somme de l'iso-idide estérifié, de l'isosorbide estérifié et de l'isomannide estérifié présent dans la composition est dans la plage de 40:60 à 65:35, de préférence de 45:55 à 60:40,
et/ou
- le rapport molaire iso-idide non estérifié : iso-idide estérifié présent dans la composition est dans la plage de 80:20 à 95:5,
et/ou
- le rapport molaire isosorbide non estérifié éventuellement présent : isosorbide estérifié éventuellement présent dans la composition est dans la plage de 20:80 à 0,01:99,99, de préférence de 5:95 à 0,5:99,5,
et/ou
- le rapport molaire isomannide non estérifié éventuellement présent : isomannide estérifié éventuellement présent dans la composition est dans la plage de 20:80 à 0,01:99,99, de préférence de 5:95 à 0,1:99,9,
et/ou
- la composition comprend de l'iso-idide, du 2,5-diacétate d'isomannide et du monoacétate d'isosorbide, de préférence le 5-monoacétate d'isosorbide.

12. Composition telle que définie dans l'une quelconque des revendications 10 à 11, obtenue ou pouvant être obtenue par un procédé selon l'une quelconque des revendications 1 à 9.

13. Utilisation d'une composition telle que définie dans l'une quelconque des revendications 10 à 12
- dans un procédé de fabrication et/ou d'isolement d'iso-idide, de préférence en tant que matériau de départ ou en tant qu'intermédiaire ;
et/ou
- dans un procédé de séparation de l'iso-idide d'autres dianhydrohexitols, de préférence d'un mélange d'autre dianhydrohexitols ou d'isomères de dianhydrohexitol comprenant l'isosorbide et/ou l'isomannide,
de préférence en tant que matériau de départ ou en tant qu'intermédiaire.

14. Utilisation d'un procédé selon l'une quelconque des revendications 1 à 9 comprenant une étape d'estérification sélective d'un mélange d'isomères de dianhydrohexitol comprenant de l'iso-idide, pour séparer l'iso-idide dudit mélange d'isomères de dianhydrohexitol, de préférence d'un mélange de composés de formule II tel que défini dans la revendication 1 ou 4,
dans laquelle, de préférence, le procédé comprend l'estérification sélective des isomères de dianhydrohexitol autres que l'iso-idide et ensuite la séparation de l'iso-idide non estérifié du mélange d'isomères de dianhydrohexitol estérifiés.

15. Procédé de fabrication d'un polymère comprenant des monomères d'iso-idide ou des monomères d'iso-idide modifiés, le procédé comprenant la fabrication ou l'isolement des monomères d'iso-idide et/ou les monomères d'iso-idide modifiés par un procédé selon l'une quelconque des revendications 1 à 9 ou mettant en œuvre un procédé selon l'une quelconque des revendications 1 à 9.
